# EUROPEAN PATENT SPECIFICATION

(11) **EP 3 369 419 B1**
(45) Date of publication and mention of the grant of the patent: **28.08.2024**
(21) Application number: 16859957.9
(22) Date of filing: 28.10.2016
(51) Int. Cl.: A61K 31/155, A61K 31/198, A61P 3/10, A61K 38/28, A61K 45/06

(54) **COMPOSITION FOR TREATING DIABETES**
ZUSAMMENSETZUNG ZUR BEHANDLUNG VON DIABETES
COMPOSITION POUR LE TRAITEMENT DU DIABÈTE

(30) Priority: 30.10.2015 JP 2015214824
(43) Date of publication of application: 05.09.2018
(73) Proprietor: Kato, Yasumasa, Yokohama-shi, Kanagawa 221-0865 (JP)
(72) Inventor: Kato, Yasumasa, Yokohama-shi, Kanagawa 221-0865 (JP)
(74) Representative: Grünecker Patent- und Rechtsanwälte PartG mbB
(86) International application number: PCT/JP2016/082066
(87) International publication number: WO 2017/073729

(56) References cited:
- WO-A1-2005/049006
- WO-A1-2011/051974
- WO-A1-2013/134736
- WO-A1-2016/049236
- JP-A- 2004 002 272
- JP-A- 2007 008 814
- US-A1- 2013 017 283
- JAMES HEAF: "METFORMIN IN CHRONIC KIDNEY DISEASE: TIME FOR A RETHINK", vol. 34, 1 June 2014 (2014-06-01) - 1 June 2014 (2014-06-01), pages 353 - 357, XP055507219, Retrieved from the Internet <URL:https://www.ncbi.nlm.nih.gov/pmc/articles/PMC4079480/pdf/pdi_34_4_007.pdf> [retrieved on 20180914]
- SARAH VECCHIO ET AL: "Metformin-induced lactic acidosis: no one left behind", CRITICAL CARE, vol. 15, no. 1, 1 January 2011 (2011-01-01), GB, pages 107 - 108, XP055507170, ISSN: 1364-8535, DOI: 10.1186/cc9404
- LIZHI FU ET AL.: "Interaction between metformin and leucine in reducing hyperlipidemia and hepatic lipid accumulation in diet-induced obese mice", METABOLISM, vol. 64, no. 11, 17 July 2015 (2015-07-17), pages 1426 - 1434, XP055379137

## Description

### [Technical field]

The present invention relates to a composition for use in a method of treating diabetes mellitus, wherein the composition comprises a combination of active ingredients, wherein the composition has an excellent hypoglycemic effect that suppresses lactic acidosis without substantially increasing the blood lactate concentration, wherein the composition comprises the active ingredients:
a) branched-chain amino acids or derivatives of the branched-chain amino acids, and
b) biguanide derivatives, or salts of the biguanide derivatives,
wherein the biguanide derivatives are selected from the group consisting of metformin hydrochloride, phenformin and buformin, and wherein the disease to be treated is diabetes mellitus associated with renal dysfunction.

Any references in the description to methods of treatment are to be interpreted as references to the compounds and compositions of the present invention for use in those methods.

### [Background Technology]

Hypoglycemic agents are one of the conventional treatments used for diabetic patients. Oral hypoglycemic agents such as insulin preparation, sulfonylurea drugs, thiazolidinedione derivatives, alpha-glucosidase inhibitors, and biguanide agents are mainly used (for example, Patent Document I).

### Preceding technical documents

### [Patent Documents]

Vecchio S. et al., "Metformin-induced lactic acidosis: no one left behind", Critical Care, GB, published on 21 January 2011, pages 107 - 108, relates to metformin-induced lactic acidosis.

[Patent document 1] Japanese Unexamined Patent Application Publication No. 2007-008814.

### [Summary of the invention]

### [Problems to be resolved by the invention]

Among oral hypoglycemic agents, the possibility of biguanide derivatives causing lactic acidosis is recognized to be high. For example, existing biguanide agents such as metformin are associated with the risk of causing lactic acidosis among diabetic patients with a history of lactic acidosis, diabetic patients with renal dysfunction, diabetic patients with liver dysfunction, diabetic patients with cardiovascular disorders, diabetic patients with impaired pulmonary function, diabetic patients who are prone to hypoxemia, diabetic patients who consume excess alcohol, diabetic patients with gastrointestinal disorders, and elderly diabetic patients.

The present invention intends to solve the above problem by providing a composition for use in a method of treating diabetes mellitus having excellent hypoglycemic effect that suppresses lactic acidosis without substantially increasing the blood lactate concentration, as defined in the appended claims.

### [Means for Solving Problem]

To achieve the above objective, the present invention provides a composition for use in a method of treating diabetes mellitus with hypoglycemic effect that suppresses lactic acidosis without substantially increasing the blood lactate concentration, and is characterized by a branched-chain amino acid or a derivative of a branched-chain amino acid, and biguanide derivative or a salt of the biguanide derivative as the active component, as defined in the appended claims.

The composition for treating diabetes mellitus of the present invention may contain either leucine, isoleucine, or valine, or both leucine and isoleucine, or, all of leucine, isoleucine, and valine as the branched-chain amino acid.

The composition for treating diabetes mellitus of the present invention may contain metformin, phenformin, or buformin as the biguanide derivative.

The disease to be treated by the composition for treating diabetes mellitus of the present invention is

The target disease of the present invention is diabetes mellitus associated with renal dysfunction and the invention can also be used for the prevention and treatment of lactic acidosis.

The present invention can be in the form of infusion or oral preparations.
1. Supplementation and enhancement of the treatment and preventive effectiveness of the composition,
2. Improvement of the dynamics and absorption of the composition, reduction in dosage, and
3. May be administered as a drug combined with other drugs to alleviate side effects caused by the composition.

For example, can be administered as a drug in combination with Branched-Chain Amino Acids (BCAA), biguanide derivatives such as metformin or salts of the biguanide derivatives and derivatives of branched-chain amino acids or therapeutic agents used in the treatment of diabetes mellitus.

The above-mentioned therapeutic agents used in the treatment of diabetes mellitus include but are not limited to dipeptidyl peptidase-4 inhibitors (hereinafter abbreviated as "DPP4 inhibitors"), sulfonylurea hypoglycemic drugs, biguanide preparations, α-glucosidasc inhibitors, rapid-acting insulin secrctagogues, insulin preparations, PPAR agonists, *β*3 adrenergic receptor agonists, aldose reductase inhibitors, GLP-1 analogs, and SGLT inhibitors.

DPP4 inhibitors include vildagliptin, P-32/98, P-93/01, TS-021, 815541, 825964, denagliptin, TA-6666, MK-0431/ONO-5435, SYR-322, SK-042, saxagliptin, and KRP-104.

The drug containing the composition of the present invention may be administered as a combination drug in which all the components are combined in one formulation or administered in the form of separate formulations. Administration as separate formulations includes simultaneous and separate administrations. Also, when administering separately, the compound of the present invention may be administered first, and the other drug may be administered later, or the other drug may be administered first, and the compound of the present invention may be administered later, and respective mode of administration may be the same or different.

### [Effect of the invention]

As described above, according to the composition for use in a method of treating diabetes mellitus of the present invention, a therapeutic agent with excellent hypoglycemic effect that suppresses lactic acidosis without substantially increasing the blood lactate concentration is provided, as defined in the appended claims.

DPP4 inhibitors are present in new drugs of diabetes mellitus and use of drugs formulated by combining DPP4 inhibitors and metformin has started recently, and there is widespread recognition that blood glucose cannot be controlled without using a biguanide derivative like metformin. The discovery of the fact that BCAA (Branched-chain Amino Acid) suppresses the side effects of metformin and increases hypoglycemic effect is considered to be very important for the future where metformin is expected to be used frequently.

The efficacy of diabetes mellitus drugs containing biguanide derivatives like metformin will be examined. According to our current knowledge, pancreas regains its activity after resting. Metformin lowers blood glucose by suppressing the manufacture of glucose in the liver, this allows the pancreas to rest without getting tired, and is considered to promote activation of the pancreas. Also, the hypoglycemic effect of metformin is remarkable when used in the early stage of diabetes mellitus. Addition of BCAA to metformin further enhances the hypoglycemic effect and the drug can also be administered without problems to older adults who require caution during administration.

Population aging is expected to progress throughout the world in the future, and elderly diabetic patients are also expected to increase. The number of patients with kidney failure undergoing hemodialysis will also increase leading to higher medical expenses. The composition for treating diabetes mellitus of the present invention with active components metformin and BCAA is expected to be good news not only for Japan but also for countries and people all over the world.

### [Brief Description of the Drawings]

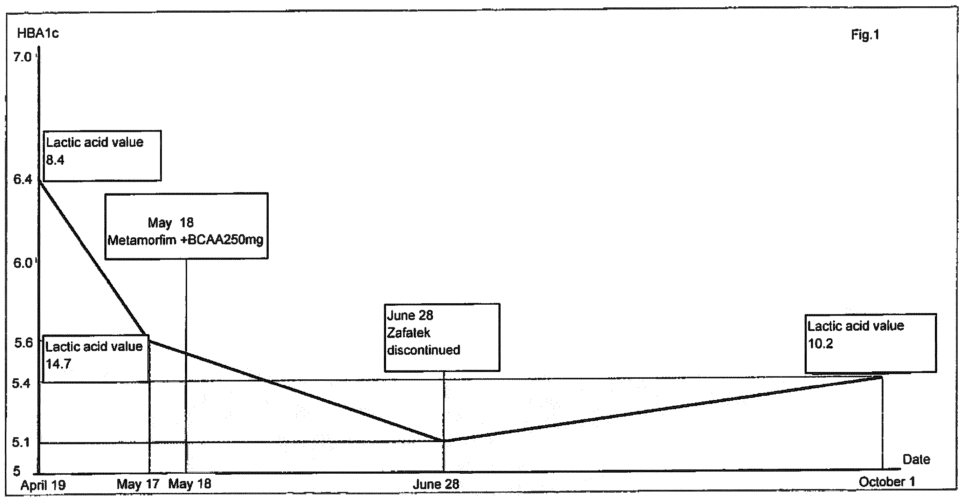

[Fig. 1] is a graph showing the transition of HbA1c for a patient undergoing metformin + BCAA therapy when the new drug (DPP4 inhibitor: Zafatek) is used independently or in combination.

### [Mode for carrying out the claimed invention]

### (Composition for treating diabetes mellitus)

A preferred embodiment of the present invention will be described in detail. The composition for treating diabetes mellitus of the present invention with hypoglycemic effect that suppresses lactic acidosis without substantially increasing the blood lactate concentration, has a branched-chain amino acid or a derivative of a branched-chain amino acid, and biguanide derivative or a salt of the biguanide derivative as the active component, as defined in the appended claims.

The drug containing the composition of the present invention may be administered as a combination drug in which both the components are combined in one formulation or administered in the form of separate formulations. Administration as separate formulations includes simultaneous and separate administrations. Also, when administering separately, the compound of the present invention may be administered first, and the other drug may be administered later, or the other drug may be administered first, and the compound of the present invention may be administered later, and respective mode of administration may be the same or different.

Each component constituting the therapeutic agent for diabetes mellitus of the present invention will be described. First, the composition for treating diabetes mellitus of the present invention contains a branched-chain amino acid or a derivative of a branched-chain amino acid as one of the active components.

The form of branched-chain amino acids include but are not limited to pure crystalline amino acids in free form, and their salts, peptides, or derivatives. Branched-chain amino acid in the salt form include pharmacologically acceptable salt forms such as sodium, potassium, hydrochloride, and acetate salts. Branched-chain amino acid in the peptide form include peptides obtained by peptidcization of branched-chain amino acid as a dipeptide or tripeptide. Peptideizing branched-chain amino acid in this way enables peptides to be effectively utilized after conversion into free amino acids by hydrolysis with the in vivo action of peptidase. Derivatives of branched-chain amino acids include N-acetyl-DL-leucine, DL-norleucine, N-acetyl-DL-isoleucine, 4-hydroxy-L-isoleucine, and *β*-methyl norleucine. These derivatives are converted to free amino acids by the in vivo effect of enzymes such as acylase and can be used effectively.

The composition for treating diabetes mellitus of the present invention may contain either leucine, isoleucine or valine, or both leucine and isoleucine, or, all of leucine, isoleucine, and valine as the branched-chain amino acid.

Leucine, isoleucine, and valine are compounds represented by the respective chemical formulas given below.

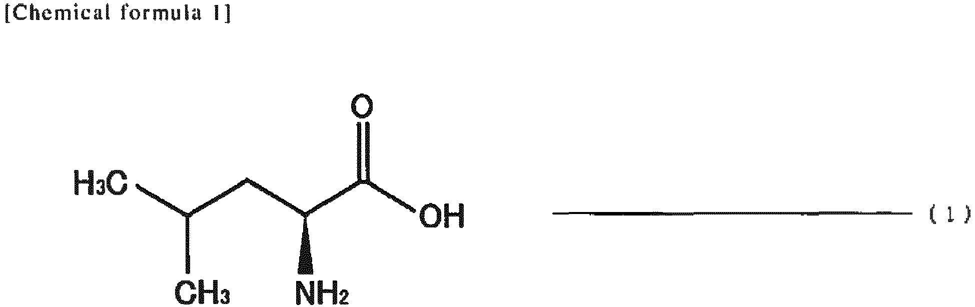

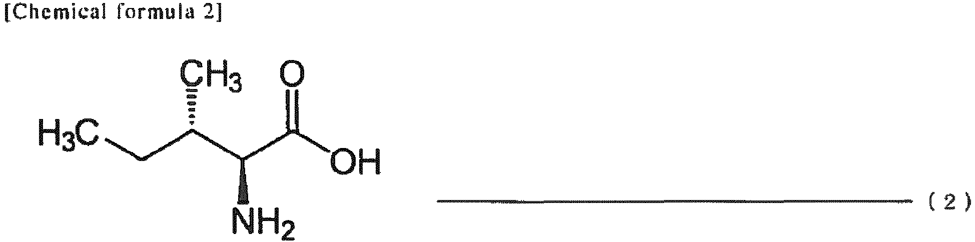

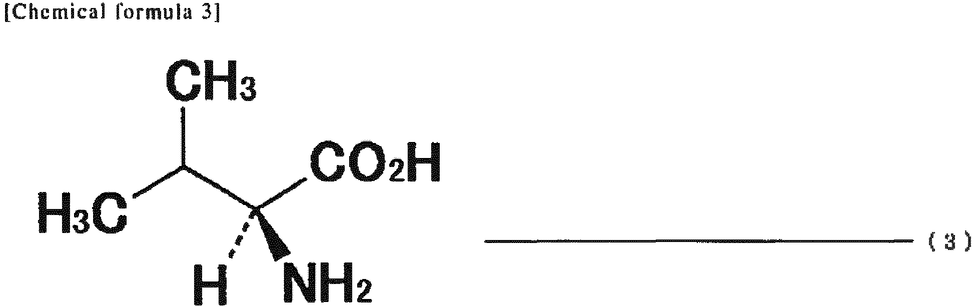

The composition for treating diabetes mellitus of the present invention may contain all of leucine, isoleucine, and valine as the branched-chain amino acid. When the composition for treating diabetes mellitus containing both leucine and isoleucine as branched-chain amino acids is specifically used as a preparation, then leucine, isoleucine, and valine will not have an antagonistic action on the albumin production effect of each other, and additive effectiveness of in vivo production promoting effect of albumin can be improved.

The composition for treating diabetes mellitus of the present invention may contain only one of leucine, isoleucine, or valine as the branched-chain amino acid. The composition for treating diabetes mellitus containing leucine, isoleucine, or valine independently will further decrease the in vivo load of proteins, effectively reducing side effects when the composition for treating diabetes mellitus is used as a preparation.

For example, if the emphasis is on the efficacy during administration of the composition for treating diabetes mellitus to patients with liver diseases, both leucine and isoleucine must be included in the composition for treating diabetes mellitus to improve the additive effect of the production promoting effect of albumin described above. On the other hand, if the emphasis is on the safety during administration of the composition for treating diabetes mellitus to patients with liver diseases, the composition for treating diabetes mellitus must include leucine or isoleucine to effectively reduce the side effects caused by the reduction of the protein load as described above. That is, the composition for treating diabetes mellitus can achieve a balance between efficacy and safety based on the condition of patients with liver diseases.

On the other hand, the composition for treating diabetes mellitus of the present invention contains a biguanide derivative or a salt of the biguanide derivative as one of the active components.

The salt of the biguanide derivative must be a pharmacologically acceptable salt, the salts include, for example, salts with inorganic acid, salts with organic acid, and salts with an acidic amino acid. The salts with inorganic acids include, for example, salts with hydrochloric acid, hydrobromic acid, nitric acid, sulfuric acid, and phosphoric acid. The salts with organic acids include, for example, salts with formic acid, acetic acid, trifluoroacclic acid, fumaric acid, oxalic acid, tartaric acid, maleic acid, citric acid, succinic acid, malic acid, methanesulfonic acid, benzenesulfonic acid, and p-toluenesulfonic acid. The salts with acidic amino acid include, for example, salts with aspartic acid and glutamic acid.

Specifically, metformin hydrochloride, represented by the following chemical formula (4), 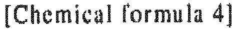 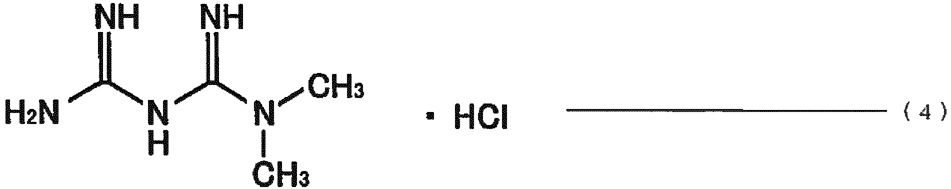 as a salt of the biguanide derivative can be included as one of the active components.

Also, biguanide derivatives buformin and phenformin are compounds represented by the following chemical formula [I] or [II], rcspectively.

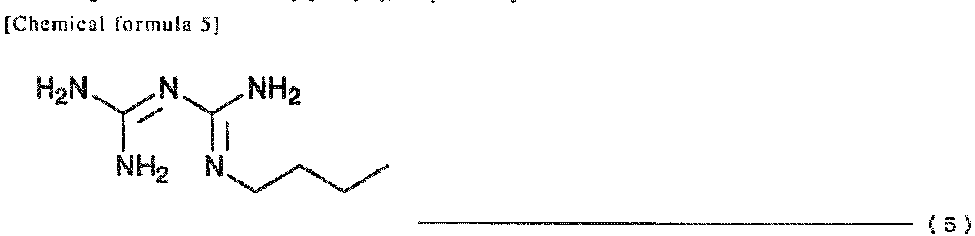

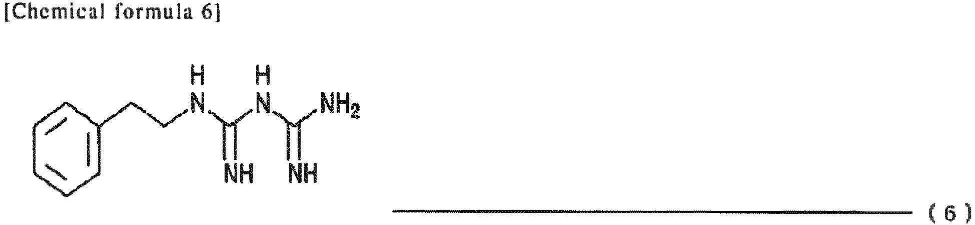

Moreover, pharmaceutically acceptable salts of buformin or phenformin can be used including salts with inorganic acids such as hydrochloric acid, hydrobromic acid, hydroiodic acid, nitric acid, sulfuric acid, phosphoric acid; salts with organic acids such as acetic acid, fumaric acid, maleic acid, succinic acid, citric acid, tartaric acid, adipic acid, gluconic acid, glucoheptonic acid, glucuronic acid, terephthalic acid, methancsulfonic acid, lactic acid, hippuric acid, 1,2-ethancdisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, and sulfosalicylic acid; quaternary ammonium salt with methyl bromide, and methyl iodide; salts with halogen ions such as bromine ion, chlorine ion, and iodine ion; salts with alkali metals such as lithium, sodium, and potassium; salts with alkaline earth metals such as calcium, and magnesium; metal salts with iron and zinc; salt with ammonia; salts with organic amines such as triethylenediamine, 2-aminocthanol, 2,2'-Iminobis(ethanol), 1-deoxy-1-(methylamino)-2-D-sorbitol, 2-amino-2-(hydroxymethyl)-1,3-propanediol, procaine, and N,N'-bis (phenylmethyl)-1,2-ethanediamine, but hydrochloride is preferred. Moreover, buformin or phenformin may take the form of a hydrate or a solvate.

When geometric or optical isomers are present in buformin or phenformin, their isomers or salts are also included in the scope of the present invention. When proton tautomers exist in buformin or phenformin, their tautomers or salts are also included in the scope of the present invention.

When crystalline polymorph and crystalline polymorph group (crystalline polymorph system) exists in buformin and phenformin or their geometric isomers, optical isomers, proton tautomers or salts, then their crystalline polymorphs and crystalline polymorph groups (crystalline polymorph system) are also included in the scope of the present invention. Here, the crystalline polymorph group (crystalline polymorph system) refers to the individual crystals formed at each stage when the crystal form changes based on the conditions and state (including the state of the formulation) such as production, crystallization, preservation of these crystals, and the entire process.

The composition for treating diabetes mellitus of the present invention may contain the branched-chain amino acid or derivative of a branched-chain amino acid, and biguanide derivative or salt of the biguanide derivative as the active component, and the specific formulation may be prepared, for example, by mixing with excipients, binders, stabilizers, lubricants, flavoring agents, disintegrating agents, coating agents, coloring agents, buffering agents, aqueous solvents, oily solvents, tonicity agents, dispersants, preservatives, solubilizing agents, fluidizing agents, soothing agents, pH adjusting agents, antiseptics, and bases. Also, physiologically permissible carriers can be used as additive components of the composition for treating diabetes mellitus.

Excipients include sugars such as lactose, sucrose, glucose, D-mannitol, and sorbitol; cellulose such as crystalline cellulose, hydroxypropyl cellulose, hydroxypropyl methylcellulose, methylcellulose, and their derivatives; starch such as corn starch, potato starch, α-starch, dextrin, *β*-cyclodextrin, sodium carboxymethyl starch, hydroxypropyl starch, and their derivatives; silicates such as synthetic aluminum silicate, magnesium aluminosilicate, calcium silicate, and magnesium silicate; phosphates such as calcium phosphate, carbonates such as calcium carbonate; sulfates such as calcium sulfate; tartaric acid, potassium hydrogen tartrate, and magnesium hydroxide.

Binders include cellulose such as crystalline cellulose, hydroxypropyl cellulose, hydroxypropyl methylcellulose, methylcellulose and their derivatives; starch such as corn starch, potato starch, α-starch, dextrin, *β*-cyclodextrin, sodium carboxymethyl starch, hydroxypropyl starch, and their derivatives; sugars such as lactose, sucrose, glucose, D-mannitol, and sorbit; agar, stearyl alcohol, gelatin, tragacanth, polyvinyl alcohol, and polyvinylpyrrolidone.

Stabilizers include p-hydroxybenzoic esters such as methyl paraben and propyl paraben; alcohols such as chlorobutanol, benzyl alcohol, and phenylethyl alcohol; phenols such as phenol and cresol; sulfites such as sodium bisulfite and sodium sulfite; edetate salts such as disodium edetatc, and tetrasodium edetate; hydrogenated oil, sesame oil, sodium chondroitin sulfate, dibutyl hydroxy toluene, adipic acid, ascorbic acid, L-ascorbyl stearate, sodium L-ascorbate, L-aspartic acid, monosodium L-aspartatc, sodium acetyltryptophanate tryptophan, acetanilide, aprotinin solution, aminoethylsulfonic acid, aminoacetic acid, DL-alanine, L-alanine, benzalkonium chloride, and sorbic acid.

Lubricants include stearic acids such as stearic acid, calcium stearate, and magnesium stearate; waxes such as white beeswax and carnauba wax; sulfate such as sodium sulfate; silicates such as magnesium silicate and light anhydrous silicic acid; lauryl sulfate such as sodium lauryl sulfate; powdered acacia, cocoa butter, carmellose calcium, carmellose sodium, callopeptide, hydrated silicon dioxide, hydrated amorphous silicon oxide, dried aluminum hydroxide gel, glycerin, light liquid paraffin, crystalline cellulose, hydrogenated oil, synthetic aluminum silicate, sesame oil, wheat starch, talc, macrogols, and phosphoric acid.

Flavoring agents include sugars such as lactose, sucrose, glucose, and D-mannitol; ascorbic acid, L-aspartic acid, L-ascorbyl stearate, monosodium L-aspartate, magnesium L-aspartate, aspartame, sweet hydrangea leaf, sweet hydrangea leaf extract, powdered sweet hydrangea leaf, aminoethylsulfonic acid, aminoacetic acid, DL-alanine, sodium saccharin, DL-menthol, and L-menthol.

Disintegrating agents include cellulose such as crystalline cellulose, hydroxypropyl cellulose, hydroxypropyl methylcellulose, methylcellulose, and their derivatives; carbonates such as calcium carbonate, sodium bicarbonate, and magnesium carbonate; starch such as corn starch, potato starch, α-starch, dextrin, *β*-cyclodextrin, sodium carboxymethyl starch, hydroxypropyl starch, and their derivatives; agar, gelatin, tragacanth, adipic acid, alginic acid, and sodium alginate.

Coating agents include cellulose derivatives such as cellulose acetate, hydroxypropyl cellulose, cellulose acetate phthalate, and hydroxypropyl methylcellulose: shellac, polyvinylpyrrolidones, polyethylene glycol, macrogols, methacrylic acid copolymers, liquid paraffin, and eudragit. Coloring agents include indigo carmine, caramel, and riboflavin.

Buffering agents include aminoacetic acid, L-arginine, benzoic acid, sodium benzoate, ammonium chloride, potassium chloride, sodium chloride, dried sodium sulfite, dried sodium carbonate, diluted hydrochloric acid, citric acid, calcium citrate, sodium citrate, disodium citrate, calcium gluconate, L-glutamic acid, L monosodium glutamate, creatinine, chlorobutanol, crystalline sodium dihydrogen phosphate, disodium succinate, acetic acid, potassium acetate, sodium acetate, tartaric acid, sodium bicarbonate, sodium carbonate, triethanolamine, lactic acid, sodium lactate solution, glacial acetic acid, boric acid, maleic acid, anhydrous sodium citrate, anhydrous sodium acetate, anhydrous sodium carbonate, anhydrous disodium hydrogen phosphate, anhydrous trisodium phosphate, anhydrous sodium dihydrogen phosphate, DL-malic acid, phosphoric acid, trisodium phosphate, sodium hydrogen phosphate, dipotassium phosphate, potassium dihydrogen phosphate, sodium dihydrogen phosphate, and sodium dihydrogen phosphate monohydrate.

Aqueous solvents include distilled water, physiological saline, and Ringer's solution. Oily solvents include vegetable oils such as olive oil, sesame oil, cottonseed oil, and corn oil; and propylene glycol. Tonicity agents include potassium chloride, sodium chloride, glycerin, sodium bromide, D-sorbitol, nicotinamide, glucose, and boric acid.

Dispersants include stearic acid and its salts such as zinc stearate and magnesium stearate; acacia, propylene glycol alginate, sorbitan sesquioleate, D-sorbitol, tragacanth, methylcellulose, aluminum monostearate, aminoalkyl methacrylate copolymer RS, lactose, concentrated glycerin, propylene glycol, macrogols, and sodium lauryl sulfate.

Preservatives include alcohols such as chlorobutanol, phenethyl alcohol, propylene glycol, and benzyl alcohol; p-hydroxybenzoic esters such as isobutyl parahydroxybenzoate, ethyl parahydroxybenzoate and methyl parahydroxybenzoate; benzalkonium chloride, benzethonium chloride, dried sodium sulfite, cresol, chlorocrcsol, dibutyl hydroxy toluene, potassium sorbate, sodium dehydroacetale, phenol, formalin, phosphoric acid, benzoin, thimerosal, thymol, and sodium dehydroacetate.

Solubilizing agents include sodium benzoate, ethylenediamine, citric acid, sodium citrate, glycerin, sodium acetate, sodium salicylate, sorbitan sesquioleate, nicotinamide, glucose, benzyl alcohol, polyvinylpyrrolidones, acetone, ethanol, isopropanol, D-sorbitol, sodium bicarbonate, sodium carbonate, lactose, urea, and sucrose.

Fluidizing agents include stearic acid and its salts such as calcium stearate and magnesium stearate; hydrated silicon dioxide, talc, anhydrous ethanol, crystalline cellulose, synthetic aluminum silicate, and calcium phosphate. Soothing agents include benzalkonium chloride, procaine hydrochloride, meprylcaine hydrochloride, lidocaine hydrochloride, and lidocaine.

pH adjusting agents include hydrochloric acid, citric acid, succinic acid, acetic acid, boric acid, maleic acid, and sodium hydroxide. Antiseptics include benzoic acid, sodium benzoate, cetylpyridinium chloride, salicylic acid, sodium salicylate, sorbic acid, potassium sorbate, thymol, methyl parahydroxybenzoate, and butyl parahydroxybenzoate.

Bases include vegetable oils such as olive oil, sesame oil, and wheat-germ oil; glycerin, stearyl alcohol, polyethylene glycols, propylene glycol, cetanol, lard, white petrolatum, paraffin, bentonite, isopropyl lanolate, Vaseline, polysorbates, macrogols, lauryl alcohol, sodium lauryl sulfate, ethyl linoleate, sodium hydrogen phosphate, and rosin.

There is no specific restriction on the dosage form of the composition for treating diabetes mellitus of the present invention, granules, powders, tablets, capsules, syrups, emulsions and suspensions are some of the different forms; some of the parenteral agents are injections such as subcutaneous injection, intravenous injection, intramuscular injection, and intraperitoneal injections; transdermal administration agents such as ointments, creams, and lotions; suppositories such as rectal and vaginal suppositories; nasal administration formulations. Various preparations mentioned above can be produced by known methods commonly used in the preparation process.

Next, the specific effect of the composition for treating diabetes mellitus of the present invention "Hypoglycemic effect without substantially increasing the blood lactate concentration" will be described.

In the present invention, "Hypoglycemic effect without substantially increasing the blood lactate concentration" indicates that when the composition for treating diabetes mellitus is administered and hypoglycemic fall rate is measured with oral glucose tolerance test and blood lactate concentration is measured, the increase rate of blood lactate concentration is 35% or less at the dose at which the hypoglycemic fall rate is 60 to 80%, while the preferable increase rate of blood lactate concentration is 30% or less at the dose at which the hypoglycemic fall rate is 60 to 80%, and increase rate of blood lactate concentration of 25% or less at the dose at which the hypoglycemic fall rate is 60 to 80% is further preferred. That is, for example, when hypoglycemic fall rate is measured with oral glucose tolerance test and blood lactate concentration is measured, blood lactate concentration is suppressed to 45 mg/dl or less even when the composition for treating diabetes mellitus is administered with a dose at which the hypoglycemic fall rate indicates 60 to 80% for diabetic patients with blood lactate concentration indicated to be between 4 to 33 mg before administration of the composition.

Next, diabetic patients who can be considered for administration of the composition for treating diabetes mellitus of the present invention will be described.

Since the composition for treating diabetes mellitus of the present invention has hypoglycemic effect without substantially increasing the blood lactate concentration as described above, the composition is effective especially for diabetic patients who are prone to develop lactic acidosis. Such diabetic patients who tend to develop lactic acidosis includes, for example, diabetic patients with a history of lactic acidosis, diabetic patients with renal dysfunction, diabetic patients with liver dysfunction, diabetic patients with cardiovascular disorders, diabetic patients with impaired pulmonary function, diabetic patients who are prone to hypoxemia, diabetic patients who consume excess alcohol, diabetic patients with gastrointestinal disorders, patients with type 2 diabetes, and elderly diabetic patients.

The composition for treating diabetes mellitus of the present invention is specifically effective for diabetic patients who tend to develop lactic acidosis as described above, and even among these patients, the composition for treating diabetes mellitus is suitable for administration to diabetic patients with renal dysfunction. Renal dysfunction includes chronic renal failure, diabetic nephropathy, glomerulonephritis, immune complex nephritis, acute renal failure, interstitial nephritis, nephrosclerosis, renal infarction, renal tubule dysfunction, renal impairment due to drugs, renal impairment due to pesticides, and uremia.

Next, the administration method of the composition for treating diabetes mellitus of the present invention will be described.

The administration method of the composition for treating diabetes mellitus of the present invention, for example, can be but are not limited to peroral or parenteral administration as a pharmaceutical composition (formulation) using branched-chain amino acid or derivatives of branched-chain amino acids, biguanide derivatives or salts of the biguanide derivatives and the above mentioned additive components.

The dosage of the composition for treating diabetes mellitus of the present invention can be appropriately determined based on the type of target (warm-blooded animals such as human beings), severity of symptoms, age, administration method, and results of diagnosis by doctors, but for example, the following administration dosages of biguanide derivatives are preferred for an adult, 0.1 to 2000 mg/kg per day in the case of oral administration, and 0.1 to 1000 mg/kg per day is preferred in the case of parenteral administration. The dosage given above is the value per unit weight (body weight of 1 kg) of the administration target. The dosage of the present invention mentioned above may be administered at once or divided into several doses over a period of 1 to 7 days depending on the severity of symptoms and diagnosis of the doctor.

### (Form of the preparations)

The composition for treating diabetes mellitus can be suitably used in the form of a preparation. The preparation forms include but are not limited to infusion preparations, oral preparations, transdermal absorption preparations, suppositories, patches, ointments, haps, and lotions.

The composition for treating diabetes mellitus can be suitably used in the form of infusion preparation. By preparing the composition for treating diabetes mellitus in the form of infusion preparation, the composition for treating diabetes mellitus can be administered quickly and effectively through the blood vessels, and maximum efficacy in promoting the in vivo albumin production can be demonstrated.

Infusion preparation types include injections and drip infusions. When the composition for treating diabetes mellitus is used as an injection or drip infusion, they should be sterilized and made isotonic with blood. When preparing the composition for treating diabetes mellitus as an injection or drip infusion, diluting agents that can be used for example include water, ethyl alcohol, polyethylene glycol, propylene glycol, ethoxylated isostearyl alcohol, polyoxylated isostearyl alcohol, and polyoxyethylene sorbitan fatty acid esters, and sufficient quantity of salt, glucose, or glycerin can be added to prepare a solution that is isotonic with body fluids. The infusion preparation can be cryopreserved, or can also be stored by removing moisture by lyophilization. The infusion preparation that has been stored after lyophilization can be dissolved by adding distilled water for injections or sterilized water when required to be used.

The composition for treating diabetes mellitus can be suitably used in the form of oral preparations. By preparing the composition for treating diabetes mellitus in the form of oral preparations, the composition for treating diabetes mellitus can be administered easily and conveniently without invasion into the living organism, and adequate effect in promoting the in vivo albumin production can be demonstrated.

The oral preparations include but are not limited to tablets, powders, granules, fine granules, pills, capsules, lozenges, chewables, and syrups. When used as tablets, various carriers known in the field of hypoalbuminemia improvement can be used. Carriers include excipients such as lactose, sucrose, sodium chloride, glucose, urea, starch, calcium carbonate, kaolin, crystalline cellulose, and silicic acid; Binders such as water, ethanol, propanol, simple syrup, glucose solution, starch solution, gelatin solution, carboxymcthylcellulose, shellac, methylcellulose, potassium phosphate, and polyvinylpyrrolidone; disintegrating agents such as dry starch, sodium alginate, powdered agar, laminaran powder, sodium bicarbonate, calcium carbonate, polyoxyethylene sorbitan fatty acid esters, sodium lauryl sulfate, stearic acid monoglyceride, starch, and lactose; Disintegrating inhibitors such as sucrose, stearin, cocoa butter, and hydrogenated oil; Absorption enhancers such as quaternary ammonium base, and sodium lauryl sulfate; moisturizers such as glycerin, and starch; Adsorbents such as starch, lactose, kaolin, bentonite, and colloidal silicic acid; Lubricants such as purified talc, stearate, boric acid powder, and polyethylene glycol. Such tablets may include, as necessary, tablets with a normal coating, sugar-coated tablets, gelatin-coated tablets, enteric coated tablets, film-coated tablets, double-layered tablets, and multi-layered tablets.

When used as a pill, various carriers known in the field of hypoalbuminemia improvement are used. Carriers include excipients such as glucose, lactose, starch, cocoa butter, hydrogenated vegetable oil, kaolin, and talc; Binders such as powdered acacia, tragacanth powder, gelatin, and ethanol; Laminaran, and agar.

The above mentioned oral preparation may further contain additives. Such additives include surfactants, absorption enhancers, fillers, bulking agents, moisturizers, antiseptics, stabilizers, emulsifiers, solubilizers, and salts for regulating osmotic pressure, and can be used by selecting based on the form for administration unit of the oral preparation.

The composition for treating diabetes mellitus of the present invention is not limited to the above embodiment. When the form of the composition for treating diabetes mellitus of the present invention is in the form of an oral preparation, a paste (thickeners, and gelling agents) may be added as necessary to prepare the composition in the form of a gel or as jelly. By adjusting the composition for treating diabetes mellitus of the present invention in the form of a gel or as jelly, oral administration becomes easy and absorption in the gastrointestinal tract also improves. The type of pastes include but are not limited to agar, gelatin, carrageenan, Arabic gum, guar gum, locust bean gum, tara gum, gellan gum, curdlan, xanthan gum, pullulan, pectin, sodium alginate, carboxymethylcellulose, other polysaccharides that can be usually used as a paste, and one or two or more types of these can be used in combination. The proportion of such types of paste should preferably be not more than five parts by mass for 100 parts by mass of the composition for treating diabetes mellitus that is prepared in the form of a gel or as jelly.

### (Therapeutic agents used in the treatment of diabetes mellitus such as DPP4 inhibitors)

1. Supplementation and enhancement of the treatment and preventive effectiveness of the composition,
2. Improvement of the dynamics and absorption of the composition, reduction in dosage, and
3. May be administered as a drug combined with other drugs to alleviate side effects caused by the composition. For example, can be administered as a drug in combination with Branched-chain Amino Acids (BCAA), biguanide derivatives such as metformin or salts of the biguanide derivatives and derivatives of branched-chain amino acids or therapeutic agents used in the treatment of diabetes mellitus.

The above-mentioned therapeutic agents used in the treatment of diabetes mellitus include but are not limited to dipeptidyl peptidase-4 inhibitor (hereinafter abbreviated as "DPP4 inhibitor"), sulfonylurea drugs, biguanides, α-glucosidase inhibitors, insulin secretagogues, insulin sensitizers, insulin preparations, PPAR agonists (PPARα agonists, PPARy agonists, PPARα + y agonists, and PPAR pan agonists), *β*3 adrenergic receptor agonists, aldose reductase inhibitors, AMP kinase activator, 11*β*-hydroxysteroid dehydrogenase (11*β*-HSD1) type I inhibitor, lipase inhibitors, and appetite suppressants.

The drug containing the composition of the present invention may be administered as a combination drug in which both the components arc combined in one formulation or administered in the form of separate formulations. Administration as separate formulations includes simultaneous and separate administrations. Also, when administering separately, the compound of the present invention may be administered first, and the other drug may be administered later, or the other drug may be administered first, and the compound of the present invention may be administered later, and respective mode of administration may be the same or different. The diseases for which therapeutic and prophylactic effect is obtained with the combination drug include but are not limited to any disease that supplements and enhances the therapeutic and preventive effect of the compound of the present invention.

DPP4 inhibitors include LAF-237, sitagliptin phosphate (MK-431, ONO-5435), BMS-477118, P93-01, GSK823093, GSK815541, GSK825964, TS-021, T-6666, SYR -322, E-3024, NN-7201, and PSN-9301.

Sulfonylurea drugs include acetohexamide, glibenclamide, gliclazide, glyclopyramide, chlorpropamide, tolazamide, tolbutamide, and glimepiride.

Biguanide drugs include buformin hydrochloride and metformin hydrochloride.

α-glucusidasc inhibitors include acarbose, voglibose, and miglitol.

Insulin secretagogues include nateglinide, repaglinide, and mitiglinide. Insulin sensitizers include ONO-5816, YM-440, JTT-501, and NN-2344.

PPAR agonists include bezafibrate, clofibrate, fenofibrate, and gemfibralc that are PPAR α agonists, pioglitazone, troglitazone, and rosiglitazone that are PPAR y agonists, muraglitazar, tesaglitazar, and ONO-5129 that are PPAR α+γ agonists, and GSK 677954, PLX 204, and MCC-555 that are PPAR pan agonists.

*β*3 adrenergic receptor agonists include AJ9677, L750355, and CP331648.

Aldose reductase inhibitors include epalrestat, fidarestat, and zenarestat.

An example of a lipase inhibitor is orlistat.

Appetite suppressants include cannabinoid receptor I antagonists (for example, rimonabant), melanin-concentrating hormone receptor antagonists (for example, GSK856464, ATC-0065, ATC-0175, and AMG-076), monoamine oxidase inhibitors (For example, mazindol and sibutramine), serotonin 2c receptor agonists (for example, APD-356, and SCA-136), histamine 3 receptor antagonists (for example, ABT-239, ABT-837, GT-2331, and NNC-0038-0000-1202), mazindol, and sibutramine.

DPP4 is a dipeptidyl peptidase IV that is also called DPP-IV, DP4, DPPIV, and CD26; this dipeptidyl peptidase IV is a serine protease that produces dipeptide Xaa-Pro or Xaa-Ala from a peptide chain having proline or alanine at the second position from the N end. DPP4 is widely distributed in mammalian tissues and is known to be present in kidney, liver, intestinal epithelium, placenta, and blood plasma, and is involved in the metabolism of various biologically active peptides. Among them, the powerful ability of DPP4 to promote insulin secretion and play the role as an enzyme for inactivating in vivo Glucagon-Like Peptide-I (hereinafter abbreviated as GLP-I) that is responsible for regulating postprandial blood glucose has attracted attention.

In the same way as GLP-1, Gastric Inhibitory Polypeptide or Glucose-dependent Insulinotropic Peptide (referred to as GIP), Pituitary Adenylate Cyclase Activating Polypeptide (referred to as PACAP), and Vasoactive Intestinal Polypeptide; (referred to as VIP) are biologically active peptides that promote insulin secretion from the pancreas. DPP4 is also involved in the degradation of these biologically active peptides due to which compounds that inhibit DPP4 also suppresses degradation of these biologically active peptides, enhances action and insulin secretion, and is expected to be useful in prevention and treatment of diabetes mellitus (especially type 2 diabetes and the like) or in prevention and improving postprandial hyperglycemia, and impaired glucose tolerance.

DPP4 is also involved in the metabolism of neuropeptides such as neuropeptide Y, endomorphin 1, endomorphin 2, and substance P. Therefore, compounds that inhibit DPP4 can also be expected to be used as therapeutic agents or analgesics for schizophrenia, depression, anxiety, epilepsy, and stress-related diseases as they suppress the degradation of biologically active peptides.

DPP4 is known to be involved in the metabolism of various cytokines and chcmokines, activation of T cells which are immunocompetent cells, adhesion of cancer cells to the endothelium, and proliferation of blood cells. Since compounds inhibiting DPP4 also inhibit these actions, they are useful for the treatment and prevention of autoimmune diseases such as rheumatoid arthritis and type I diabetes, allergic diseases such as asthma and food allergies, cancer, cancer metastasis, HIV infection, anemia, and thrombocytopenia.

Since high expression of DPP4 is found in skin fibroblasts of patients with psoriasis, rheumatoid arthritis and lichen planus, and high DPP4 activity is found in patients with prostatic hyperplasia, compounds that inhibit DPP4 are also expected to be effective in treating and preventing skin diseases (psoriasis, and lichen planus) and prostatic hyperplasia.

In addition to the above, compounds inhibiting DPP4 are also considered to be useful in treating and preventing hyperlipidemia, metabolic syndrome (syndrome X), diabetic complications, arteriosclerosis, polycystic ovary syndrome, infertility, growth disorders, arthritis, transplant rejection, enteritis, and trauma.

### [Example of execution]

The present invention will be described in detail with a formulation example, but the present invention is not intended to be limited to this formulation example alone.

In the prescription examples below, along with providing treatment based on the administration of metformin hydrochloride and branched-chain amino acids such as L-isoleucine, L-leucine, and L-valine (hereinafter referred to as "Metformin + BCAA therapy"), a general therapeutic agent used in the treatment of diabetes mellitus such as DPP4 inhibitor is used in combination, depending on the condition of the patient. For each prescription example, the glycemic status of the patient that is dependent on the administered active components and the component amount is measured using HbA1c (glycated hemoglobin) along with changes in the lactic acid value, and opinion of the doctor is recorded together with the change in the values.

### (Prescription example 1)

| | Component type | Active component name | Dosage | Administration interval | Observation period |
|---|---|---|---|---|---|
| Biguanide derivatives etc. | | Metformin hydrochloride | 125 mg/administration | 1 time/day | 1 month |
| Branched-chain amino acids etc. | | L-Isolcucinc | 952 mg/administration | | |
| | | L-Leucine | 1904 mg/administration | | |
| | | L-valine | 1144 mg/administration | | |

**[Table 1]**

| | | | | | |
|---|---|---|---|---|---|
| Gender | Female | | | | |
| Age | 99 years | | | | |
| Prescription period | June 13, 2015 | HbA1c | 6.6 mg/dl | | |
| | July 18, 2015 | HbA1c | 5.6 mg/dl | Lactic acid value | 8.1 |
| | July 29, 2015 | HbA1c | 5.5 mg/dl | | |
| | August 17, 2015 | | | Lactic acid value | 15.9 |
| | September 4, 2015 | | | Lactic acid value | 12.1 |
| | September 5, 2015 | HbA1c | 5.1 mg/dl | | |
| | November 5, 2015 | | | Lactic acid value | 17.2 |

| | | | | | |
|---|---|---|---|---|---|
| o Opinion of the doctor | | | | | |

The patient was an older adult and was suffering from renal dysfunction, but HbA1c level dropped by 1.0 (approximately 15%) in around one month, the patient regained appetite with the decrease in blood glucose level, and there were no side effects. The lactic acid value at the end of the observation period also stabilized to a normal value at 8.1 (<16.0).

In the United States, Cr is a measure of renal dysfunction, and use of metformin is prohibited in men who have a Cr of 1.5 or more and women who have a Cr of 1.4 or more because the possibility for lactic acid values to increase is high (There are no special regulations in Japan). In this prescription example, a 99-year-old female patient was provided treatment for about 5.5 months from June 13, 2015, to November 29, 2015. During her first visit, HbAlc was 6.6, and she was diagnosed with renal anemia. On June 27, Hb was 5.5 and Ht was 17.3.

Metformin hydrochloride 250 mg was prescribed on June 15, 2015. Cr was 1.67 on July 29, 2015. BCAA2P was added on July 30, 2015. On August 17, 2015, the lactic acid value was 15.9. Since HbAlc level dropped to 5.1 on September 5, 2015, the dose of metformin was reduced from 250 mg to 125 mg.

On November 9, 2015, the HbA1c level was 5.2, the lactic acid value was 20.9 and Cr was 1.65, but the administration of metformin was discontinued on November 18, 2015. The values recorded were the last as the patient died on November 29. Anemia due to impaired function of bone marrow and renal impairment associated with anemia are the main ailments of senility. The cause of death was not lactic acidosis due to metformin. Lactic acidosis can be considered as the cause of death if the lactic acid value exceeds 40.

Another example is of a 97-year-old female patient who had little consciousness when she was brought to me, and the lactic acid value was 41.9 on July 2, 2016, she gradually regained consciousness about a month later, on August 9, 2016, the lactic acid value returned to a normal value 7.3, and she could understand when others spoke to her (she used to take deep breaths during stethoscope examination, and stick her tongue out). Though there is a 2-year age difference between the patients who are 99 and 97 years old, this case serves as a reference to the fact that even if the lactic acid value exceeds 41, lactic acidosis does not become a direct cause of death.

The result of this prescription example is that metformin can be used in combination with BCAA even at the advanced age of 99 when Cr is more than 1.4, and renal failure is so severe that transfusion or use of renal hormone (erythropoietin) is inevitable. Even the patient who died did not require dialysis.

### (Prescription example 2)

Metformin was used in combination with BCAA (Branched-chain Amino Acid) to treat a diabetic patient who was using insulin since the age of 17.

| Component type | Active component name | Dosage | Administration interval | Observation period |
|---|---|---|---|---|
| Biguanide derivatives etc. | Metformin hydrochloride | 250 mg/administration | 3 times/day | 1 month |
| Branched-chain amino acids etc. | L-isoleucine | 952 mg/administration | | |
| | L-Leucine | 1904 mg/administration | | |
| | L-valine | 1144 mg/administration | | |

**[Table 2]**

| Gender | Female | | |
|---|---|---|---|
| Age | 41 years | | |
| Prescription period | August 12, 2015 | Lactic acid value | 5.9 |
| | August 26, 2015 | Lactic acid value | 4.7 |
| | September 12, 2015 | Lactic acid value | 3.6 |

| | | | |
|---|---|---|---|
| o Opinion of the doctor | | | |

Although blood glucose level did not decrease, the lactic acid value dropped from 5.9 to 3.6.

### (Prescription example 3)

The patient was not able to eat meals after having become bedridden and was administered high-caloric infusion, following this the blood glucose level gradually increased. One tablet of metformin (250 mg) and BCAA for hypoalbuminemia was prescribed.

| Component type | Active component name | Dosage | Administration interval | Observation period |
|---|---|---|---|---|
| Biguanide derivatives etc. | Metformin hydrochloride | 125 mg/administration | 1 day | 1 month |
| Branched-chain amino acids etc. | L-Isoltucinc | 952 mg/administration | | |
| | L-Leucine | 1904 mg/administration | | |
| | L-valine | 1144 mg/administration | | |

**[Table 3]**

| | | | | | |
|---|---|---|---|---|---|
| Gender | | | Female | | |
| Age | 89 years | | | | |
| Prescription period | July 7, 2015 | HbA1c | 6.2 mg/dl | | |
| | August 5, 2015 | HbA1c | 5.7 mg/dt | Lactic acid value | 10.8 |

| | | | | | |
|---|---|---|---|---|---|
| o Opinion of the doctor | | | | | |

HbAlc level had decreased by 0.5 mg/dl (about 8%) in one month, and metformin was set at 125 mg/day as the level sometimes dropped too low during this period. Bringing borderline diabetes mellitus to the normal value range is relatively difficult, but the lactic acid value also reached the normal range (<16.0) without side effects, even though the patient was an older adult.

### (Prescription example 4)

Metformin (250 mg) x 1 to 2 tablets were prescribed in combination with insulin treatment for a patient was bedridden due to hypoxic encephalopathy resulting from hypoglycemia.

| Component type | Active component name | Dosage | Administration interval | Observation period |
|---|---|---|---|---|
| Biguanide derivatives etc. | Metformin hydrochloride | 250 mg/administration | 2 times/day | 13 months |
| Branched-chain amino acids etc. | L-Isoleucine | 952 mg/administration | | |
| | L-Leucine | 1904 mg/administration | | |
| | L-valine | 1144 mg/administration | | |

**[Table 4]**

| Gender | Male | | | | |
|---|---|---|---|---|---|
| Age | 68 years | | | | |
| Prescription period | October 29, 2013 | HbA1c | 7.6 mg/dl | | |
| | March 16, 2014 | HbA1c | 5.9 mg/dl | (Reference value 6.2) | |
| | June 6, 2015 | HbA1c | 5.9 mg/dl | Lactic acid value | 5.1 |

| | | | | | |
|---|---|---|---|---|---|
| o Opinion of the doctor | | | | | |

The HbAlc level which was 7.6 mg/dl during first prescription decreased to 5.9 mg/dl (approximately 20%) and the latest lactic acid value during the observation period was stable at 5.1 (<16.0), a normal value.

### (Prescription example 5)

| Component type | Active component name | Dosage | Administration interval | Observation period |
|---|---|---|---|---|
| Biguanide derivatives etc. | Metformin hydrochloride | 250 mg/administration | 3 times/day | 6 months |
| Branched-chain amino | L-Isoleucine | 952 mg/administration | | |
| acids etc. | L-Leucine | 1904 mg/administration | | |
| | L-valinc | 1144 mg/administration | | |

**[Table 5]**

| Gender | Male | | | | |
|---|---|---|---|---|---|
| Age | 49 years | | | | |
| Prescription period | March 13, 2014 | HbA1c | 9.2 mg/dl | | |
| | December 8, 2014 | HbA1c | 11.1 mg/dl | Lactic acid value | 16.3 |
| | March 18, 2015 | HbA1c | 8.6 mg/dl | | |
| | April 1, 2015 | HbA1c | 8.2 mg/dl | Lactic acid value | 9.3 |
| | June 12, 2015 | HbA1c | 7.4 mg/dl | Lactic acid value | 12.3 |

| | | | | | |
|---|---|---|---|---|---|
| o Opinion of the doctor | | | | | |

HbAlc value decreased from 8.6 mg/dl to 7.4 mg/dl (approximately 14%) in 2 months from the beginning of 2015 when BCAA and metformin (250 mg) x 6 tablets were given in combination with insulin treatment, and the HbAlc value was approximately 30% lower than the maximum value of 11.1 mg/dl. Lactic acid value also stabilized at 12.3, a normal value.

### (Prescription example 6)

Metformin and BCAA was prescribed for the treatment of diabetes mellitus.

| Component type | Active component name | Dosage | Administration interval | Observation period |
|---|---|---|---|---|
| Biguanide derivatives etc. | Metformin hydrochloride | 250 mg/administration | 2 times/day | 16 months |
| Branched-chain amino acids etc. | L-Isoleucine | 952 mg/administration | | |
| | L-Leucine | 1904 mg/administration | | |
| | L-valine | 1144 mg/administration | | |

**[Table 6]**

| Gender | Female | | | | |
|---|---|---|---|---|---|
| Age | 74 years | | | | |
| Prescription period | March 27, 2014 | HbA1c | 9.4 mg/dl | | |
| | July 10, 2014 | HbA1c | 6.1 mg/dl | | |
| | March 28, 2015 | | | Lactic acid value | 6.1 |
| | June 6, 2015 | HbAlc | 5.6 mg/dl | | |

| | | | | | |
|---|---|---|---|---|---|
| o Opinion of the doctor | | | | | |

In the initial stage, HbAlc level decreased from 9.4 mg/dl to 6.1 mg/dl (approximately 35%) in a brief period from March to July 2014, and in a period of one year and few months including the above period, HbAlc level decreased by approximately 40%, from 9.4 mg/dl to 5.6 mg/dl. As of June 2015, lactic acid value also stabilized at a normal value of 9.6 (<16.0).

### (Prescription example 7)

| Component type | Active component name | Dosage | Administration interval | Observation period |
|---|---|---|---|---|
| Biguanide derivatives etc. | Metformin hydrochloride | 250 mg/administration | 1 time/day | 1 month |
| Branched-chain amino acids etc. | L-Isoleucine | 952 mg/administration | | |
| | L-Leucine | 1904 mg/administration | | |
| | L-valine | 1144 mg/administration | | |

**[Table 7]**

| | | | | | |
|---|---|---|---|---|---|
| Gender | Male | | | | |
| Age | 76 years | | | | |
| Prescription period | September 11, 2015 | HbA1c | 7.0 mg/dl | | |
| | October 4, 2015 | HbA1c | 6.3 mg/dl | | |
| | October 16, 2015 | | | Lactic acid value | 14.2 |

| | | | | | |
|---|---|---|---|---|---|
| o Opinion of the doctor | | | | | |

In the initial stage, the HbAlc level decreased from 7.0 mg/dl to 6.3 mg/dl (approximately 10%) in a brief period from September to October 2015, and in October 2015 lactic acid value was also stable at 14.2 (< 16.0).

### (Prescription example 8)

| Component type | Active component name | Dosage | Administration interval | Observation period |
|---|---|---|---|---|
| Biguanide derivatives etc. | Metformin hydrochloride | 250 mg/administration | 2 times/day | 9 months |
| Branched-chain amino acids etc. | L-Isoleucine | 952 mg/administration | | |
| | L-Leucine | 1904 mg/administration | | |
| | L-valine | 1144 mg/administration | | |

**[Table 8]**

| | | | | | |
|---|---|---|---|---|---|
| Gender | Male | | | | |
| Age | 75 years (diabetes mellitus, dementia) | | | | |
| Prescription period | October 14, 2015 | HbA1c | 8.6 mg/dl | Lactic acid value | 18.6 |
| | June 29, 2016 | HbA1c | 7.9 mg/dl | | |
| | July 4, 2016 | | | Lactic acid value | 15.1 |

| | | | | | |
|---|---|---|---|---|---|
| o Opinion of the doctor | | | | | |

HbAlc level was 8.6 and lactic acid value was 18.6 > 16.0 from the test results during the first visit (October 14, 2015) of the patient and was administered oral antidiabetic drugs (5 types, one of which was 500 mg of metformin) along with administration of insulin (5 units) for 24 hours.

The patient was in a state where glycemic control was not possible. The uric acid value was 18.6 (> Standard value 16) indicating that the dose of metformin could not be increased any further. Metformin (+ BCAA also) was increased for treatment of the patient. Diarrhea, the second side effect of metformin set in and administration of drugs to stop diarrhea did not yield any results. However, diarrhea could be stopped by significantly reducing the lipid intake and this is a measure for the second side effect of metformin.

The efficacy of metformin, a drug for diabetes mellitus will be discussed According to our knowledge, pancreas regains its activity after resting. Metformin lowers blood glucose by suppressing the manufacture of glucose in the liver, this allows the pancreas to rest and is considered to promote activation of the pancreas. Also, the hypoglycemic effect of metformin is remarkable when used in the early stage of diabetes mellitus. Addition of BCAA to metformin further enhances the hypoglycemic effect and can also be administered without problems to older adults who require caution during administration.

Population aging is expected to progress throughout the world in the future, and elderly diabetic patients are also expected to increase. The number of patients with kidney failure undergoing hemodialysis will also increase leading to higher medical expenses. Both metformin and BCAA of the present invention are expected to be good news not only for Japan but also for countries and people all over the world.

Glycemic control of the patient in this example was almost successful under the following conditions. I am saying "Almost successful" because stabilizing HbA1c level to a lower value is possible, but the function of the pancreas was considered to have declined due to aging, and bringing the HbA1c level below the standard value of 6.2 was likely to be difficult.

Thus, a little more time is required to stabilize blood glucose levels of patients for whom glycemic control is not possible (especially older adults). Approximately eight months was required to reach the following state. For this reason, administration of the composition for treating diabetes mellitus of the present invention which contains both metformin and BCAA as the active components must be started at an early stage of diabetes. This patient had started to develop necrosis of the left leg because of the above therapy, but at present, the symptoms cannot be observed at all.

HbAlc level for this patient on June 29, 2016, was 7.9 and lactic acid value on July 4, 2016, was 15.1. The patient was administered with metformin 1000 mg, pioglitazone (was taking from the first visit) an oral drug that docs not burden the pancreas, and 5 to 8 units of insulin with effect lasting for 24 hours.

This patient was referred to our hospital by a different hospital, and the patient exercised in a wheelchair underwent rehabilitation for dementia and was on a 1600 kcal/day diet. Since dementia had advanced, and the patient was bedridden during our initial examination, we reduced the diet to 1200 kcal/day. This difference of 400 kcal can be the energy that is almost equal or higher to the exercise done by the patient in the wheelchair.

### (Prescription example 9)

| Component type | Active component name | Dosage | Administration interval | Observation period |
|---|---|---|---|---|
| Biguanide derivatives etc. | Metformin hydrochloride | 250 mg/administration | 2 times/day | 4 months |
| Branched-chain amino acids etc. | L-Isoleucine | 952 mg/administration | | |
| | L-Leucine | 1904 mg/administration | | |
| | L-valine | 1144 mg/administration | | |

**[Table 9]**

| | | | |
|---|---|---|---|
| Gender | Male | | |
| Age | 52 years | | |
| Prescription period | February 24, 2016 | HbA1c | 9.1 mg/dl |
| | June 25, 2016 | HbA1c | 7.6 mg/dl |

| | | | |
|---|---|---|---|
| o Opinion of the doctor | | | |

This case with improvement of not only HbAlc but also the liver enzyme that is involved in fatty liver (non-alcoholic) was added to the results of metformin + BCAA therapy of the composition for treating diabetes mellitus of the present invention.

### (Prescription example 10)

| Component type | Active component name | Dosage | Administration interval | Observation period |
|---|---|---|---|---|
| Biguanide derivatives etc. | Metformin hydrochloride | 250 mg/administration | 2 times/day | 11 months |
| Branched-chain amino acids etc. | L-Isoleucine | 952 mg/administration | | |
| | L-Leucine | 1904 mg/administration | | |
| | L-valine | 1144 mg/administration | | |

**[Table 10]**

| | | | | |
|---|---|---|---|---|
| Gender | Male | | | |
| Age | 73 years | | | |
| Prescription period | June 11, 2015 | HbA1c...6.4 mg/dl | | |
| | August 5, 2015 | | Lactic acid value | 9.5 |
| February 5, 2016 | HbA1e...5.5 mg/dl | | | |
| July 5, 2016 | HbA1c...5.5 mg/dl | Lactic acid value | 15.1 | |

| | | | | |
|---|---|---|---|---|
| o Opinion of the doctor | | | | |

The treatment of diabetes mellitus is considered to require exercise therapy. Exercising the body to control blood glucose certainly brings down the blood glucose level.

The patient of this prescription example was already bedridden with little consciousness during the first visit. During the first visit (March 2015), he was suffering from hypoglycemia during the treatment of diabetes mellitus, developed hypoxic encephalopathy and was bedridden. He was feed 1100 kcal/day through a feeding tube. Since the patient suffered from aspiration pneumonia that was because of reflux from the stomach for about six months, he was fed using IVH (1000 kcal high-caloric infusion through the jugular vein) from September up to July 2016. Only drugs are being passed to the stomach through the nasal passage, and the patient has settled down. Medicine is drugs and metformin 500 mg + BCAA.

The values shown in the above table arc,

| | | |
|---|---|---|
| June 2015 | HbA1c 6.4 | (metformin 250 mg, lactic acid value 9.5) |
| July 2016 | HbA1c 5.5 | (lactic acid value 15.1) |

The results indicate that the composition for treating diabetes mellitus of the present invention can be used for glycemic control of a person who was not able to move his body for a year and few months after the first visit. The results also indicate that blood glucose can be controlled even though glucose was directly injected into the blood vessels with IVH procedure. A better way to control the blood glucose level is providing metformin + BCAA therapy with the composition for treating diabetes mellitus of the present invention while the diabetes mellitus condition is still not serious.

### (Prescription example 11)

| Component type | Active component name | Dosage | Administration interval | Observation period |
|---|---|---|---|---|
| Biguanide. derivatives etc. | Metformin hydrochloride | 250 mg/administration | 1 time/day (other. increased dose to 3 times/day) | 4 months |
| Branched-chain amino acids etc. | L-Isoleucine | 952 mg/administration | | |
| | L-Leucine | 1904 mg/administration | | |
| | L-valine | 1144 mg/administration | | |
| Diabetes mellitus therapy (DPP4 inhibitor) | Dipeptidyl peptidase-4 inhibitor | 100 mg/administration | 1 time /week | 1 month |

**[Table 11]**

| | | | | | |
|---|---|---|---|---|---|
| Gender | Male | | | | |
| Age | 85 years | | | | |
| Prescription period | April 19, 2016 | HbA1c | 6.4 mg/dl | Lactic acid value | 8.4 |
| | May 17, 2016 | HbA1c | 5.6 mg/dl | Lactic acid value | 14.7 |
| | June 28, 2016 | HbA1c | 5.1 mg/dl | Lactic acid value | 12.2 |
| | July 20, 2016 | HbA1c | 5.2 mg/dl | | |
| | August 24, 2016 | | | Lactic acid value | 13.3 |
| | October 1, 2016 | HbA1c | 5.4 mg/dl | Lactic acid value | 10.2 |

| | | | | | |
|---|---|---|---|---|---|
| o Opinion of the doctor | | | | | |

Fig. 1 shows the change in values of HbAlc for a patient undergoing metformin + BCAA therapy when the new drug (DPP4 inhibitor: Zafatek), a therapeutic agent for the treatment of diabetes mellitus is used independently or in combination. For this patient, HbA1c was 5.6 with only the new drug (DPP4 inhibitor) but was noticed only when administration of metformin 250 mg + BCAA was started. Glycemic control was not good with postprandial blood glucose at 246 mg/dl on May 9, 2016, and metformin + BCAA was started on May 18, 2016. HbAlc dropped to 5.1 on June 28, 2016, and the new drug was discontinued. The treatment was changed to just metformin + BCAA therapy. On July 20, 2016, HbAlc increased by 0.1 to 5.2. The patient did not have a good appetite at the beginning of hospitalization, but as on July 20, he was able to eat all meals. Glycemic control had also improved (fasting blood glucose, and postprandial blood glucose)

One of the side effects of metformin is elevated lactic acid values, but there is data to suggest that the new drug also has the same side effect. This suggests that BCAA must be used even in combination with the new drug.

HbAlc was 6.4, and the lactic acid value was 8.4 for this patient during admission (April 19, 2016), and HbAic was 5.6, and the lactic acid value was 14.7 on May 17, 2016. Administration of metformin was started from May 18, 2016. The lactic acid value was 13.3 on August 24, 2016, HbAlc was 5.4 on October 1, 2016, and an equivalent effect was observed even with the new drug.

### (Prescription example 12)

| Component type | Active component name | Dosage | Administration interval | Observation period |
|---|---|---|---|---|
| Biguanide derivatives etc. | Metformin hydrochloride | 250 mg/administration | 1 time/day | 3 months |
| Branched-chain amino acids etc. | L-Isoleucine | 952 mg/administration | | |
| | L-Leucine | 1904 mg/administration | | |
| | L-valine | 1144 mg/administration | | |

**[Table 12]**

| | | | | |
|---|---|---|---|---|
| Gender | Female | | | |
| Age | 89 years | | | |
| Prescription period | July 21, 2016 | HbA1c...6.9 mg/dl | | |
| | August 3, 2016 | HbA1c...6.9 mg/dl | | |
| | August 24, 2016 | | Lactic acid value | 8.4 |
| | September 1, 2016 | HbA1c...6.9 mg/dl | | |
| | October 1, 2016 | HbA1c...6.4 mg/dl | Lactic acid value | 9.1 |

| | | | | |
|---|---|---|---|---|
| o Opinion of the doctor | | | | |

This prescription example is of a case in which the side effects were suppressed by careful administration of the drug. During first visit (July 21, 2016), HbAlc was 6.9, and the lactic acid value was 17.9. A slightly higher dose 4 mg of antihypertensive diuretic fluitran that is administered with care to diabetic patients was used, but fluitran worsened the condition of diabetes mellitus and another antihypertensive drug was administered, and the condition of the patient was monitored.

One tablet of metformin (250 mg) and BCAA was administered on July 25, 2016, the lactic acid value was 8.4 on August 24, 2016, and HbA1c was 6.4 on October 1, 2016.

The results indicated that the lactic acid value could he reduced significantly, but mean blood glucose level could not be reduced. Significant reduction in the lactic acid value was considered to have prevented the condition of diabetes mellitus from worsening. In future, HbAlc was expected to come down by reducing or not using fluitran and adding metformin to the treatment. The dosage of fluitran was gradually reduced and replaced by another antihypertensive drug on September 27, 2016.

There were significant changes in the lactic acid values when measured repeatedly, and we came to understand that the value increases when the patient is not feeling well (such as infectious disease, anemia, fatigue, increase in GOTGPT, and elevated BUN) from the monitoring of the patient for about two years. If the lactic acid value increases during the course of treating diabetes mellitus with metformin + BCAA based on the composition for treating diabetes mellitus of the present invention, factors other than the dosage of metformin have to be considered.

### [Industrial Applicability]

As described above, according to the therapeutic agent for the treatment of diabetes mellitus of the present invention, a therapeutic agent with excellent hypoglycemic effect that suppresses lactic acidosis without substantially increasing the blood lactate concentration and at the same time can prevent the initiation of lactic acidosis can be provided. A therapeutic agent that can prevent and treat hyperglycemia without increasing the blood lactate concentration which may cause lactic acidosis can be provided to diabetic patients according to the present invention, as defined in the appended claims.

## Claims

1. A composition for use in a method of treating diabetes mellitus, wherein the composition comprises a combination of active ingredients, wherein the composition has an excellent hypoglycemic effect that suppresses lactic acidosis without substantially increasing the blood lactate concentration,
wherein the composition comprises the active ingredients:
a) branched-chain amino acids or derivatives of the branched-chain amino acids, and
b) biguanide derivatives, or salts of the biguanide derivatives,
wherein the biguanide derivatives are selected from the group consisting of metformin hydrochloride, phenformin and buformin, and
wherein the disease to be treated is diabetes mellitus associated with renal dysfunction.

2. The composition for use according to claim 1, wherein the composition further comprises a therapeutic agent, wherein the therapeutic agent preferably comprises an inhibitor of dipeptidyl peptidase-4 (DPP4).

3. The composition for use according to claim 1 or claim 2, wherein either leucine, isoleucine, or valine is included as the branched-chain amino acid.

4. The composition for use according to claim 1 or claim 2, wherein either leucine or isoleucine is included as the branched-chain amino acid.

5. The composition for use according to claim 1 or claim 2, wherein leucine, isoleucine, and valine are included as the branched-chain amino acids.

6. The composition for use according to claim 1 or claim 2, wherein the composition is in the form of an infusion preparation.

7. The composition for use according to claim 1 or claim 2, wherein the composition is in the form of an oral preparation.

## Patentansprüche

1. Zusammensetzung zur Verwendung in einem Verfahren zur Behandlung von Diabetes mellitus, wobei die Zusammensetzung eine Kombination von aktiven Inhaltsstoffen umfasst, wobei die Zusammensetzung eine hervorragende hypoglykämische Wirkung aufweist, die eine Laktatazidose verhindert, ohne die Blutlaktatkonzentration wesentlich zu erhöhen,
wobei die Zusammensetzung die folgenden aktiven Inhaltsstoffe umfasst:
a) verzweigtkettige Aminosäuren oder Derivate der verzweigtkettigen Aminosäuren, und
b) Biguanid-Derivate oder Salze der Biguanid-Derivate,
wobei die Biguanid-Derivate aus der Gruppe bestehend aus Metformin-Hydrochlorid, Phenformin und Buformin ausgewählt sind, und
wobei die zu behandelnde Erkrankung Diabetes mellitus in Verbindung mit renaler Dysfunktion ist.

2. Zusammensetzung zur Verwendung nach Anspruch 1, wobei die Zusammensetzung ferner einen therapeutisch wirksamen Stoff umfasst, wobei der therapeutisch wirksame Stoff vorzugsweise einen Inhibitor von Dipeptidylpeptidase 4 (DDP4) umfasst.

3. Zusammensetzung zur Verwendung nach Anspruch 1 oder Anspruch 2, wobei Leucin, Isoleucin oder Valin als die verzweigtkettige Aminosäure enthalten ist.

4. Zusammensetzung zur Verwendung nach Anspruch 1 oder Anspruch 2, wobei entweder Leucin oder Isoleucin als die verzweigtkettige Aminosäure enthalten ist.

5. Zusammensetzung zur Verwendung nach Anspruch 1 oder Anspruch 2, wobei Leucin, Isoleucin und Valin als die verzweigtkettigen Aminosäuren enthalten sind.

6. Zusammensetzung zur Verwendung nach Anspruch 1 oder Anspruch 2, wobei die Zusammensetzung in der Form einer Infusionszubereitung vorliegt.

7. Zusammensetzung zur Verwendung nach Anspruch 1 oder Anspruch 2, wobei die Zusammensetzung in der Form einer oralen Zubereitung vorliegt.

## Revendications

1. Composition pour une utilisation dans un procédé de traitement du diabète sucré, dans laquelle la composition comprend une combinaison d'ingrédients actifs, dans laquelle la composition a un excellent effet hypoglycémiant qui supprime l'acidose lactique sans augmenter sensiblement la concentration de lactate dans le sang.
dans laquelle la composition comprend les ingrédients actifs :
a) des acides aminés à chaîne ramifiée ou des dérivés des acides aminés à chaîne ramifiée, et
b) des dérivés de biguanide, ou des sels des dérivés de biguanide,
dans laquelle les dérivés de biguanide sont choisis dans le groupe constitué du chlorhydrate de metformine, de la phenformine et de la buformine, et
dans laquelle la maladie à traiter est le diabète sucré associé à un dysfonctionnement rénal.

2. Composition pour une utilisation selon la revendication 1, dans laquelle la composition comprend en outre un agent thérapeutique, dans laquelle l'agent thérapeutique comprend de préférence un inhibiteur de la dipeptidyl peptidase-4 (DPP4).

3. Composition pour une utilisation selon la revendication 1 ou la revendication 2, dans laquelle la leucine, l'isoleucine ou la valine est incluse comme étant l'acide aminé à chaîne ramifiée.

4. Composition pour une utilisation selon la revendication 1 ou la revendication 2, dans laquelle la leucine ou l'isoleucine est incluse comme étant l'acide aminé à chaîne ramifiée.

5. Composition pour une utilisation selon la revendication 1 ou la revendication 2, dans laquelle la leucine, l'isoleucine et la valine sont incluses comme étant les acides aminés à chaîne ramifiée.

6. Composition pour une utilisation selon la revendication 1 ou la revendication 2, dans laquelle la composition se présente sous la forme d'une préparation pour perfusion.

7. Composition pour une utilisation selon la revendication 1 ou la revendication 2, dans laquelle la composition se présente sous la forme d'une préparation orale.
